# EUROPÄISCHE PATENTSCHRIFT

(11) **EP 2 384 724 B1**
(45) Veröffentlichungstag und Bekanntmachung des Hinweises auf die Patenterteilung: **04.03.2020**
(21) Anmeldenummer: 11003053.3
(22) Anmeldetag: 12.04.2011
(51) Int. Cl.: A61F 2/46, B01F 5/00, B05C 17/005, B05C 17/015, A61B 17/88

(54) **Austragsvorrichtung für pastöse Massen**
Application device for paste masses
Dispositif de sortie pour masses pâteuses

(30) Priorität: 04.05.2010 DE 102010019224
(43) Veröffentlichungstag der Anmeldung: 09.11.2011
(73) Patentinhaber: Heraeus Medical GmbH, 61273 Wehrheim (DE)
(72) Erfinder: Vogt, Sebastian, 99092 Erfurt (DE); Büchner, Hubert, 90491 Nürnberg (DE)
(74) Vertreter: Heraeus IP

(56) Entgegenhaltungen:
- DE-U1- 9 403 923
- DE-U1- 20 015 942
- JP-A- S6 181 973
- JP-U- H0 615 748
- US-A- 2 818 999
- US-A1- 2006 208 000

## Beschreibung

Die vorliegende Erfindung betrifft eine Austragsvorrichtung für Kartuschen umfassend einen Anschluss zum Einspeisen eines unter Druck stehenden Gases, einen Grundkörper, in dem wenigstens ein Durchgang und/oder Kanal verläuft, durch den das unter Druck stehende Gas zu einem ersten Kartuschenanschlussstück zum Anschließen einer Kartusche leitbar ist, wobei das erste Kartuschenanschlussstück eine erste Öffnung zu dem Durchgang und/oder dem vorderen Kanal umfasst, so dass bei Beaufschlagung einer im Kartuschenanschlussstück angeordneten Kartusche mit dem unter Druck stehenden Gas, ein Kartuscheninhalt aus der Kartusche austreibbar ist.

Gegenstand der Erfindung ist also eine Austragsvorrichtung zum Auspressen von fließfähigen Massen aus Kartuschen. Die Austragsvorrichtung ist insbesondere für das Austragen von Knochenzementen bestimmt. Das Auspressen von Kartuschen mit Hilfe von komprimierten Gasen ist seit Jahrzehnten bekannt.

Die US 2,818,899 schlägt eine Dichtungspistole vor, die im Griffstück eine Gaspatrone enthält. Das komprimierte Gas der Gaspatrone drückt nach Öffnung der Patrone einen Kolben innerhalb einer Kartusche in Richtung Kartuschenkopf. Die Steuerung des Flusses der pastösen Masse erfolgt durch einen zentralen durch die Kartusche gehenden Stab, der die Austrittsöffnung der Kartusche verschließen kann.

In der US 3,938,709 von 1976 wird eine Austragsvorrichtung beschrieben, bei der durch Gasdruck eine Tube, die sich innerhalb des hohlen Pistolenkörpers befindet, ausgedrückt wird. Der Gasfluss wird dabei durch ein einfaches Stiftventil mit Feder erreicht, das durch einen Handhebel betätigt werden kann. Eine Vorrichtung zum Ablassen des Gases war dabei nicht vorhanden. Das bedeutet, dass trotz der Unterbrechung der Gaszufuhr die Pistole das Material durch den bestehenden Restdruck nachdrückt.

Die EP 0169 533 A2 von 1985 offenbart ein Einspritzgerät für viskose Mittel. Bei diesem fährt nach Unterbrechung der Druckgaszufuhr das Auspressen nicht fort, weil an der Abgabeöffnung ein Einspritzsteuerventil angeordnet ist, das den Strom des viskosen Mittels unterbricht. Interessant ist dabei, dass durch das Ventil des Abzugsgriffs einmal die Gaszufuhr und gleichzeitig der Austritt des viskosen Mittels gesteuert werden kann. Das Einspritzsteuerventil schließt, wenn keine Beaufschlagung mit komprimiertem Gas erfolgt.

In der US 4,925,061 wird ein ähnliches System beschrieben. Bei dem jedoch das Einspritzsteuerventil über eine Stange betätigt wird, die mit dem Abzugsgriff verbunden ist.

Die EP 1 118 313 A1 offenbart eine Pistole zum Auspressen von Knochenzement. Der Antrieb erfolgt hierbei ebenfalls durch eine Gaspatrone. Wesentlich ist, dass dieses sehr komplexe System eine Stange aufweist, die dazu bestimmt ist, die im Austragsrohr enthaltene Zementrestmenge auszutreiben. Diese elegante technische Lösung ist für konventionelle Polymethylmethacrylat-Knochenzemente sehr gut geeignet. Für Kartuschensysteme zur Mischung mehrerer Komponenten mit statischem Mischer ist diese Pistole jedoch nicht verwendbar. Zudem ist die Fertigung dieser Pistole sehr aufwendig.

In der US 2004/0074927 A1 wird eine Auspresspistole beschrieben, welche im Wesentlichen die gleichen Merkmale wie bei US 4,925,061 offenbart.

In den Druckschriften US 2005/0230433 A1, US 200510247740 A1 und US 6,935,541 B1 wird die im Grunde gleiche technische Lösung vorgeschlagen, die aus der EP 0 169 533 A2 bereits bekannt ist.

Die WO 2008/109439 A1 offenbart eine durch komprimiertes Gas betriebene Austragsvorrichtung, die ein Hydraulik-Medium verwendet, auf welche das komprimierte Gas drückt. Das DE 94 03 923.2 U offenbart eine Austragsvorrichtung nach dem Oberbegriff des Anspruchs 1.

Es ist festzustellen, dass die bisher bekannten Austragsvorrichtungen, die mit Gaspatronen angetrieben werden und einen komplexen mechanischen Aufbau besitzen, für die Fertigung als Einmalartikel nur bedingt oder überhaupt nicht geeignet sind. Besonders die vorgeschlagenen Ventile sind sehr teuer und stellen dadurch die Verwendung der Austragsvorrichtungen als Einmalartikel in Frage. Die vorgeschlagenen technischen Lösungen lassen sich zudem nur schwer als Kunststoffspritzteile ausführen.

Der Erfindung soll also eine einfache, unkompliziert zu handhabende und kostengünstig herzustellende Austragsvorrichtung für fließfähige Massen bereitstellen. Unter dem Begriff fließfähige Massen werden im Sinne der vorliegenden Erfindung sowohl flüssige, zähflüssige, als auch hochviskose, zähe Massen, die nur bei Druckbeaufschlagung fließen, verstanden. Das Austragen der fließfähigen Massen soll durch komprimiertes Gas erfolgen, das beispielsweise einer einsetzbaren Gaskartusche oder Druckgaspatrone entnommen wird, die sich in der Vorrichtung befindet. Diese Vorrichtung soll aus möglichst wenigen Teilen bestehen, die durch kostengünstiges Spritzgießen mit konventionellen Kunststoffen hergestellt werden können. Dafür ist es erforderlich, ein unkompliziertes, einfaches Ventilsystem zu entwickeln, das eine Kontrolle des Gasflusses innerhalb einer Verwendungszeit der Austragsvorrichtung von maximal 5 Minuten ermöglicht Die Austragsvorrichtung soll mit wenigen Handgriffen montierbar sein und unkompliziert in großen Stückzahlen hergestellt werden können. Angestrebt wird weiterhin, dass die Austragsvorrichtung als einmal zu gebrauchender Wegwerfartikel geeignet ist. Vorteilhaft wäre es zudem, wenn der Anwender den Betriebszustand der Austragsvorrichtung an der Außenseite erkennen könnte.

Der Erfindung liegt die Aufgabe zugrunde, dass mindestens zwei im Durchmesser unterschiedliche Kartuschen angeschlossen werden können. Dadurch wäre es möglich, pastöse Massen aus unterschiedlichen Kartuschensystemen mit nur einer Austragsvorrichtung auszupressen. Diese Eigenschaft würde wirtschaftliche Vorteile hinsichtlich der Amortisation der Austragsvorrichtung bringen.

Diese Aufgabe wird dadurch gelöst, dass am vorderen Rand des ersten Kartuschenanschlussstücks ein zweites Kartuschenanschlussstück mit einem größeren Querschnitt und einer zweiten Öffnung angeordnet ist, wobei das zweite Kartuschenanschlussstück zum Anschließen einer größeren Kartusche geeignet ist, und wobei die zweite Öffnung des zweiten Kartuschenanschlussstücks so groß ist, dass eine Kartusche durch die zweite Öffnung hindurch am ersten Kartuschenanschlussstück anschließbar ist.

Dabei kann vorgesehen sein, dass die Kartuschenanschlussstücke zylindrische Hohlkörper mit einer geschlossenen Seite sind, wobei die geschlossene Seite des zweiten Kartuschenanschlussstücks die zweite Öffnung umfasst und die geschlossene Seite des ersten Kartuschenanschlussstücks die erste Öffnung umfasst.

Auch wird vorgeschlagen, dass auf der Innenseite der Kartuschenanschlussstücke Fixierungsvorrichtungen zum Fixieren von Kartuschen angeordnet sind.

Dabei kann vorgesehen sein, dass die Fixierungsvorrichtungen in zwei konzentrischen Kreisen an den Kartuschenanschlussstücken angeordnet sind.

Zudem kann es vorteilhaft sein, dass die Fixierungsvorrichtungen Gewinde, insbesondere Innengewinde, sind.

Alternativ dazu kann vorgesehen sein, dass die Fixierungsvorrichtungen Rastvorrichtungen, insbesondere in Form von in eine Richtung deformierbarer Zapfen umfassen.

Erfindungsgemäße Austragsvorrichtungen können vorsehen, dass die Austragsvorrichtung einen Abzugsgriff zum Bedienen eines Ventils der Austragsvorrichtung und einen Grundkörper umfasst, in dem wenigstens ein durch das bedienbare Ventil schließbarer und zu öffnender Durchgang verläuft, durch den das unter Druck stehende Gas zu den Kartuschenanschlussstücken leitbar ist, wobei die Öffnung zu dem Durchgang führt, wobei das Ventil ein Ventilkörper ist, der drehbar im Grundkörper angeordnet ist, wobei eine Bewegung des Abzugsgriffs zu einer Drehung des Ventilkörpers im Grundkörper führt und der Durchgang im oder am Ventilkörper angeordnet ist, so dass in einer ersten Position des Ventilkörpers der Durchgang geschlossen ist und in einer zweiten Position des Ventilkörpers der Durchgang im oder am Ventilkörper das unter Druck stehende Gas zu den Kartuschenanschlussstücken leitet.

Dabei kann vorgesehen sein, dass zwischen zumindest einem Durchgang und dem Kartuschenanschlussstück zumindest ein vorderer Kanal im Grundkörper angeordnet ist und/oder zwischen zumindest einem Durchgang und dem Anschluss zum Einspeisen des unter Druck stehenden Gases zumindest ein hinterer Kanal angeordnet ist.

Ferner kann vorgesehen sein, dass ein zweiter Durchgang im oder am Ventilkörper angeordnet ist, der in einer dritten Position des Ventilkörpers die Kartuschenanschlussstücke mit einem ersten Ablass verbindet, so dass ein Überdruck an den Kartuschenanschlusstücken in der dritten Position des Ventilkörpers durch den ersten Ablass entweichen kann.

Dabei kann vorgesehen sein, dass ein erster Ablasskanal im Grundkörper derart angeordnet ist, dass er in der dritten Position des Ventilkörpers den zweiten Durchgang mit dem ersten Ablass verbindet.

Ferner wird vorgeschlagen, dass ein dritter Durchgang im oder am Ventilkörper angeordnet ist, der einen Abzweig umfasst und der in einer vierten Position des Ventilkörpers die Kartuschenanschlussstücke und das unter Druck stehende Gas mit einem zweiten Ablass verbindet, so dass ein Oberdruck in der Austragsvorrichtung in der vierten Position des Ventilkörpers durch den zweiten Ablass entweichen kann.

Dabei kann vorgesehen sein, dass zwischen dem dritten Durchgang und dem Anschluss zum Einspeisen des unter Druck stehenden Gases ein zweiter hinterer Kanal im Grundkörper angeordnet ist und/oder ein zweiter Ablasskanal im Grundkörper derart angeordnet ist, dass er in der vierten Position des Ventilkörpers den Abzweig des dritten Durchgangs mit dem zweiten Ablass verbindet.

Auch kann vorgesehen sein, dass an zumindest einem der Ablässe ein Sterilfilter angeordnet ist und/oder in zumindest einem der Ablässe ein Überdruckventil angeordnet ist.

Ferner können sich erfindungsgemäße Austragsvorrichtungen mit drehbaren Ventilkörpern dadurch auszeichnen, dass derAbzugsriff test an der unterseite aes drenbaren ventilkorpers angeordnet ist, so dass mit dem Abzugsgriff der Ventilkörper im Grundkörper drehbar ist. Solche Austragsvorrichtungen können sich auch dadurch auszeichnen, dass am drehbaren Ventilkörper ein Positionsanzeiger, insbesondere an der Oberseite, angeordnet ist, an dem die Position des Ventilkörpers im Grundkörper ablesbar ist.

Mit der Erfindung wird auch vorgeschlagen, dass an der Unterseite der Austragsvorrichtung ein Griff zum Halten der Austragsvorrichtung angeordnet ist.

Erfindungsgemäße Austragsvorrichtungen mit drehbaren Ventilkörpern können sich auch dadurch auszeichnen, dass der Ventilkörper ein konisch geformter, zumindest bereichsweise rotationssymmetrischer Körper ist, der in einer zur Form des Ventilkörpers passenden konischen Öffnung im Grundkörper drehbar gelagert ist

Dabei kann vorgesehen sein, dass die Außenwände des Ventilkörpers bis auf die Öffnungen der Durchgänge dicht mit den Wänden der konischen Öffnung im Grundkörper in jeder Position des Ventilkörpers abschließen, so dass die Verbindungen der Durchgänge zu zumindest einem Kanal im Grundkörper dicht sind.

Dabei kann ferner vorgesehen sein, dass im Grundkörper eine Ausnehmung für einen Keil vorgesehen ist, wobei ein in die Ausnehmung eingeschobener Keil den Ventilkörper in die konische Öffnung im Grundkörper presst, so dass der Ventilkörper einen gasdichten Presssitz in der konischen Öffnung erzeugt.

Weiterhin wird erfindungsgemäß vorgeschlagen, dass an der den Kartuschenanschlussstücken abgewandten Seite der Austragsvorrichtung, insbesondere im Grundkörper, ein Hohlraum für eine Gaspatrone vorgesehen ist, wobei der Anschluss zum Einspeisen eines unter Druck stehenden Gases in dem Hohlraum angeordnet ist und einen Dom zum Öffnen einer Gaspatrone umfasst, und am Grundkörper Refestigungsmittel zum Befestigen einer Verschlusskappe mit Befestigungsmitteln angeordnet sind, wobei die Gaspatrone durch das Befestigen der Verschlusskappe zu öffnen ist, wobei vorzugsweise die Verschlusskappe zusätzlich ein Arretierungsmittel umfasst, das in eine Arretierungsvorrichtung im Hohlraum greift und wobei besonders bevorzugt die Arretierungsvorrichtung eine eingesetzte Gaspatrone geeigneter Größe im Hohlraum positioniert.

Schließlich wird erfindungsgemäß vorgeschlagen, dass die Durchgänge im Wesentlichen gerade durch das Innere des Ventilkörpers verlaufen und die Durchgänge um einen Winkel von 10 ° bis 80 ° um die Drehachse des Ventilkörpers gegeneinander versetzt sind.

Der Erfindung liegt die überraschende Erkenntnis zugrunde, dass es durch das Ansetzen eines zweiten Kartuschenanschlussstücks um den Rand des ersten Kartuschenanschlussstücks herum möglich ist, einen variablen Kartuscnenanschluss bereitzustellen, so dass an die Austragsvorrichtung nicht nur eine kleine Kartusche mit nur einer Komponente als Inhalt, sondern auch ein großes Kartuschensystem für mehreren Komponenten, also eine große Mehrkomponentenkartusche angebaut beziehungsweise befestigt werden kann. Dadurch können mit der einen Austragsvorrichtung unterschiedliche Kartuschen oder Kartuschensysteme ausgepresst werden.
Ein Anschluss zum Einspeisen eines unter Druck stehenden Gases kann im Sinne der vorliegenden Erfindung ein hohler Dorn sein, auf den eine Kartusche aufgedrückt werden kann, die sich dabei öffnet. Ebenso kommt aber auch ein Rohrstück mit Gewinde als Anschluss in Frage, auf den eine Druckgaspatrone aufschraubbar ist. Auch ein Schlauchstück, der auf einen Anschluss einer Gaspatrone aufsteckbar ist, oder ein Stutzen, auf dem ein Schlauch von einem Kompressor als Druckgasquelle anschließbar ist, ist ein Anschluss zum Einspeisen eines unter Druck stehenden Gases im Sinne der vorliegenden Erfindung. Auch wenn die Druckgasquelle, beispielsweise als Teil des Grundkörpers, fest mit der Austragsvorrichtung verbunden ist, ist die Mündung der Druckgasquelle in einen Durchgang oder in einen Kanal im Grundkörper der Austragsvorrichtung ein Anschluss zum Einspeisen eines unter Druck stehenden Gases im Sinne der vorliegenden Erfindung.

Die vordere Seite ist im Sinne der vorliegenden Erfindung der Teil der Austragsvorrichtung, der zum Einsetzen der Kartusche geeignet ist. Die Kartusche wird also von Vorne in das Kartuschenanschlussstück eingesetzt. Hinten ist dementsprechend der gegenüberliegende Teil der Austragsvorrichtung. Die untere Seite der Austragsvorrichtung ist die, die im Gebrauch nach unten gehalten wird.

Quellen für das unter Druck stehende Gas können Kompressoren oder Patronen sein, wobei die Patronen mit einem komprimierten Gas, beziehungsweise einem verflüssigten Gas gefüllt sein können. Beispielsweise können Kohlendioxidpatronen verwendet werden.

Ein Sterilfilter für einen Auslass kann aus medizinischem Papier, gasdurchlässigen Kunststoffmembranen, gasdurchlässigen Kunststoffporenscheiben, gasdurchlässigen Metallporenscheiben, gasdurchlässigen Glasporenscheiben oder aus gasdurchlässigen Kunststoffvliesen gefertigt sein.

Es wird auch erfindungsgemäß vorgeschlagen, dass der Abzugsgriff U-förmig gestaltet ist. Auch kann vorgesehen sein, dass die Austragsvorrichtung im Wesentlichen aus medizinisch akzeptablen Kunstoffen gefertigt ist.

Mit der Erfindung wird auch die Verwendung einer solchen Austragsvorrichtung vorgeschlagen, zum Auspressen von fließfähigen Massen aus Kartuschen, wobei die fließfähigen Massen in den Kartuschen gelagert sind und zum Auspressen von fließfähigen Massen, die in Beuteln gelagert sind, die sich in Kartuschen befinden.

Durch den erfindungsgemäßen Aufbau ist die Ventilfunktion durch den erfindungsgemäßen Aufbau mit einem drehbaren Ventilkörper, der mit dem Grundkörper Durchgänge bildet, oder in dessen Inneren Durchgänge angeordnet sind, stark vereinfacht, um diese Funktion mit kostengünstigen Kunststoffteilen verwirklichen zu können. Das Austragen von Knochenzementen erfolgt üblicherweise im Bereich 30 Sekunden bis 5 Minuten nach dem Anmischen des Zements. Das bedeutet, das Ventil muss einen Gasstrom nur maximal 5 Minuten steuern können, die Dichtungsfunktion muss also nur wenige Minuten gewährleistet sein. Es kann angenommen werden, dass das Ventil höchstens fünfmal geöffnet und verschlossen wird. Daher ist eine derart einfache Ventilfunktion ausreichend.

Im Folgenden werden Ausführungsbeispiele der Erfindung anhand von sieben schematisch dargestellten Zeichnungen erläutert. Dabei zeigt:
- Figur 1:: eine Aufsicht auf die Seite einer erfindungsgemäßen Austragsvorrichtung;
- Figur 2:: eine Aufsicht auf die Vorderseite der erfindungsgemäßen Austragsvorrichtung nach Figur 1;
- Figur 3:: eine Detailansicht eines Fixierungselements nach Figur 1;
- Figur 4:: eine Querschnittansicht einer zweiten erfindungsgemäßen Austragsvorrichtung;
- Figur 5:: eine Quererschnittansicht eines Ventilkörpers für eine erfindungagemäße Austragsvorrichtung nach Figur 4;
- Figur 6:: eine perspektivische Ansicht des Ventilkörpers nach Figur 5; und
- Figur 7:: eine Aufsicht auf die Oberseite einer dritten erfindungsgemäßen Austragsvorrichtung.

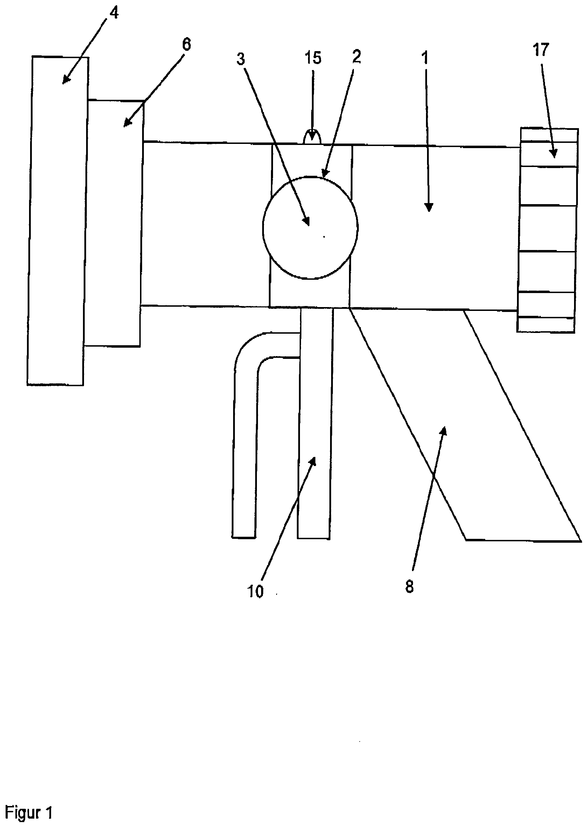

Figur 1 zeigt eine Aufsicht auf die Seite einer erfindungsgemäßen Austragsvorrichtung mit einem Grundkörper (1) mit zylindrischer Form, in dem eine seitliche Öffnung (2) in Form einer Bohrung vorgesehen ist. In der Öffnung (2) befindet sich ein in der Öffnung (2) drehbar gelagerter Ventilkörper (3) mit zylindrischer Geometrie, der mit Durchgängen im Ventilkörper (3) und im Grundkörper (1) ein Ventil bildet, so dass bei zwei unterschiedlichen Positionen des Ventilkörpers (3) zumindest zwei unterschiedliche Durchgänge durch den Ventilkörper (3) und den Grundkörper (1) geöffnet sind, während die anderen Durchgänge geschlossen sind. Die Form des Ventilkörpers (3) ist derart an die Form der Öffnung (2) angepasst, dass die Wände des Ventilkörpers (3) dicht mit der Öffnung (2) abschließen.

An der Vorderseite (in Figur 1 links) der Austragsvorrichtung ist ein erstes Kartuschenanschlussstück (4) mit einem großem Durchmesser und ein zweites Kartuschenanschlussstück (6) mit kleinerem Durchmesser angeordnet Die beiden Kartuschenanschlussstücke (4, 6) sind als zylindrische Hohlkörper ausgeformt, die auf der Vorderseite offen sind und auf der Rückseite (in Figur 1 rechts) Wände mit Öffnungen aufweisen. Die einzige Öffnung des großen Kartuschenanschlussstücks (4) ist dabei so groß, dass der Innenraum des kleinen Kartuschenanschlussstücks (6) nicht überdeckt wird. Die zumindest zwei Öffnungen in der Rückwand des kleinen Kartuschenanschlussstücks (6) führen zu den zumindest zwei Durchführungen im Grundkörper (1).

An der Unterseite der Austragsvorrichtung (in Figur 1 unten) ist ein Pistolengriff (8) angeordnet. Der Ventilkörper (3) ist an der Unterseite der Austragsvorrichtung mit einem Abzugsgriff (10) und an der Oberseite mit einem Positionsanzeiger (15) verbunden, die mit dem Ventilkörper (3) relativ zum Grundkörper (1) drehbar sind. Am Grundkörper (1) ist eine Beschriftung vorgesehen (nicht gezeigt), die dem Positionsanzeiger (15) zugeordnet ist, so dass abzulesen ist, welcher der Durchgänge durch den Ventilkörper (3) gerade geöffnet ist, beziehungsweise ob das Ventil geschlossen ist. Der Abzugsgriff (10) ist aus zwei Holmen U-förmig aufgebaut, so dass ein Anwender der Austragsvorrichtung, wenn die Finger zwischen den beiden Holmen greifen, den Abzugsgriff (10) leicht sowohl zum Pistolengriff (8) hin ziehen, als auch vom Pistolengriff (8) weg drücken kann.

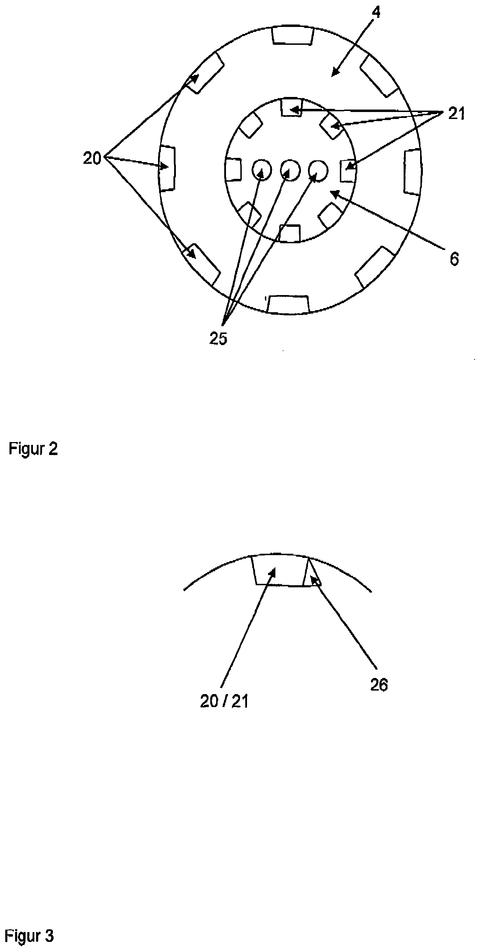

Figur 2 zeigt eine Aufsicht auf die Vorderseite der erfindungsgemäßen Austragsvorrichtung nach Figur 1. Als äußerer Ring ist das erste Kartuschenanschlussstück (4) zu erkennen, als innerer Ring das zweite Kartuschenanschlussstück (6). An den Zylinderinnenwänden der Kartuschenanschlussstücke (4, 6) sind Fixierungsvorrichtungen (20, 21) in konzentrischen Kreisen angeordnet, mit denen Kartuschen (nicht gezeigt) in den Kartuschenanschlussstücken (4, 6) fixiert werden können. Die Fixierungsvorrichtungen (20, 21) können durch Gewinde, Gewindeausschnitte oder Zapfen zum Arretieren der Kartuschen in den Kartuschenanschlussstücken (4, 6) realisiert sein.

In der rückseitigen Wand des zweiten, inneren Kartuschenanschlussstücks (6) befinden sich drei Öffnungen (25), die zu drei Durchgängen im Grundkörper (1) führen. In den Kartuschenanschlussstücken (4, 6) fixierte Kartuschen schließen dicht mit den Kartuschenanschlussstücken (4, 6) ab. Dadurch wird gewährleistet, dass ein Gasdruck, der durch eine der Öffnungen (25) auf den Boden einer fixierten Kartuschen gerichtet wird, auch auf diesen Kartuschenboden wirken kann, und nicht zwischen den Kartuschen und den Kartuschenanschlussstücken (4, 6) entweicht. Hierzu können Dichtungselemente (nicht gezeigt) in den Kartuschenanschlussstücken (4, 6) und/oder an den Kartuschen, insbesondere an den Kartuschenböden, vorgesehen sein. Es kann aber auch ausreichend sein, eine dichte Fixierungsvorrichtung (20, 21) vorzusehen, die beispielsweise durch ein Gewinde realisiert sein kann.

Figur 3 zeigt eine Fixierungsvorrichtung (20, 21) nach Figur 2, die eine Rastvorrichtung (26) umfasst, mit der eine Kartusche einem Kartuschenanschlussstück (4, 6) einrastbar ist. An den Fixierungsvorrichtungen (20, 21) zur Fixierung der Kartusche können also Rastvorrichtungen (26) in Form von in einer Richtung deformierbarer oder elastisch verformbarer Zapfen angebracht sein.

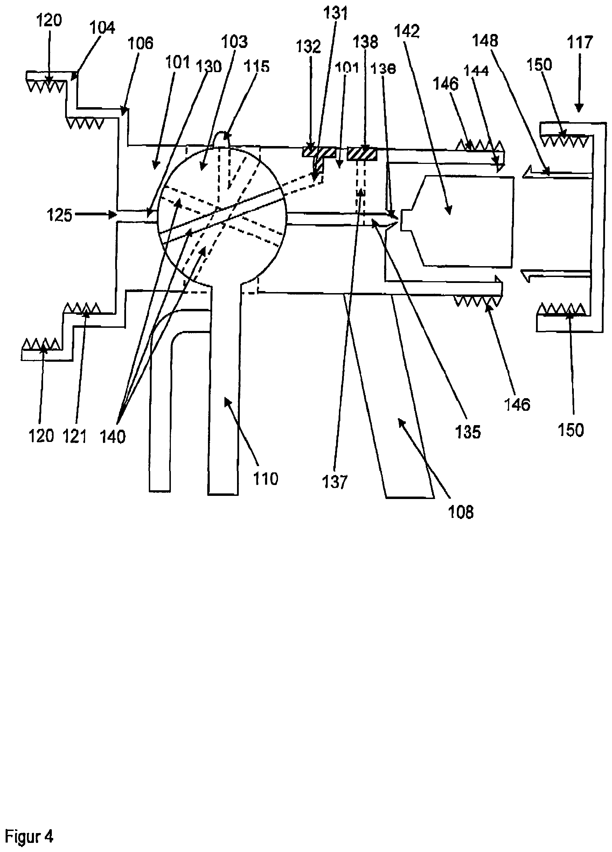

Figur 4 zeigt eine Querschnittansicht einer zweiten erfindungsgemäßen Austragsvorrichtung mit einem Grundkörper (101) an dessen Vorderseite ein erstes Kartuschenanschlussstück (104) mit großem Durchmesser für Kartuschen mit großem Durchmesser und ein zweites Kartuschenanschlussstück (106) mit kleinerem Durchmesser für Kartuschen mit kleinerem Durchmesser angeordnet sind. In einer Öffnung (nicht gezeigt) im Grundkörper (101) befindet sich ein Ventilkörper (103) mit rotationssymmetrischer Geometrie, der um seine Symmetrieachse (senkrecht zur in Figur 4 dargestellten Schnittebene) drehbar im Grundkörper (101) gelagert ist.

An der Unterseite der Austragsvorrichtung ist ein Pistolengriff (108) angeordnet, mit dem die Austragsvorrichtung mit einer Hand haltbar ist. An der Unterseite des Ventilkörpers (103) ist ein Abzugsgriff (110) zum Drehen des Ventilkörpers (103) in der Öffnung des Grundkörpers (101) angeordnet, der vor dem Pistolengriff (108) platziert ist. Der Abzugsgriff (110) ist U-förmig gestaltet, so dass die Finger einer Hand in den Zwischenraum greifen können. Die Anordnung des Abzugsgriffs (110) zum Pistolengriff (108) ist derart gewählt, dass wenn der Pistolengriff (108) in einer Hand gehalten wird, der Abzugsgriff (110) mit den Fingern derselben Hand bedienbar ist. Die U-förmige Gestaltung des Abzugsgriffs (110) erlaubt die Bewegung des Ventilkörpers (103) in beide Drehrichtungen.

Auf der Oberseite des Ventilkörpers (103) ist ein Positionsanzeiger (115) angeordnet, mit dessen Hilfe sich die Position des Ventilkörpers (103) in der Austragsvorrichtung erkennen lässt. Eine Verschlusskappe (117) ist lösbar mit der Rückseite des Grundkörpers (101) zu verbinden. Fixierungsvorrichtungen (120, 121) in Form von Innengewinden sind in den Kartuschenanschlussstücken (104, 106) zur Fixierung von Kartuschen mit Außengewinden (nicht gezeigt) vorgesehen. Im vorderen Teil des Grundkörpers (101) ist ein vorderer Kanal (130) angeordnet, der in eine Öffnung (125) zu den Kartuschenanschlussstücken (104. 106) und in eine breite Öffnung zum Ventilkörper (103) mündet.

Im hinteren Teil des Grundkörpers (101) sind drei hintere Kanäle (135) angeordnet, die in Öffnungen zum Ventilkörper (103) münden. Auf der der Rückseite der Austragsvorrichtung zugewandten Seite des Grundkörpers (101) münden zwei der drei hinteren Kanäle (135) in einen hohlen, beidseitig geöffneten Dorn (136) mit einem Durchlass. Der dritte hintere Kanal (135) mündet in einen ersten Ablasskanal (137), der über einen ersten Sterilfilter (138) mit der Umgebung der Austragsvorrichtung verbunden ist.

Im Ventilkörper (103) sind drei entlang der Symmetrieachse des Ventilkörpers (103) versetzte Durchgänge (140), die zusätzlich um diese Symmetrieachse gegeneinander verdreht sind, angeordnet. Die Durchgänge (140) sind derart im Ventilkörper (103) angeordnet und die Kanäle (130, 135) derart im Grundkörper (101) angeordnet, dass bei drei Stellungen des Ventilkörpers (103), die durch eine Drehung des Ventilkörpers (103) gegen den Grundkörper (101) einstellbar sind, der vordere Kanal (130) mit jeweils einem hinteren Kanal (135) über einen der Durchgänge (140) miteinander verbunden sind, wobei alle anderen hinteren Kanäle (135) durch den Ventilkörper (103) geschlossen sind. Die Breite der vorderen Öffnung zum Ventilkörper (103), die in den vorderen Kanal (130) mündet, reicht dazu aus, dass jeder der Durchgänge (140) mit dem vorderen Kanal (130) verbindbar ist.

Alternativ können auch drei vordere Kanäle (130) im vorderen Teil des Grundkörpers (101) vorgesehen sein, wobei jeder vordere Kanal (130) über jeweils einen Durchgang (140) mit einem hinteren Kanal (135) verbindbar ist. Jedem der drei vorderen Kanäle (130) ist dann ein hinterer Kanal (135) und ein Durchgang (140) zugeordnet. Um die Öffnungen der Kanäle (130, 135) zum Ventilkörper (103) können Dichtungen (nicht gezeigt) vorgesehen sein.

Einer der drei Durchgänge (140), die die vorderen Kanäle (130) mit den hinteren Kanälen (135) verbinden, umfasst eine Abzweigung zu einem zweiten Ablasskanal (131), der über einen zweiten Sterilfilter (132) mit der Umgebung der Austragsvorrichtung verbunden ist. Vor den Sterilfiltem (132, 138) kann ein Überdruckventil angeordnet sein, dass sich ab einem gewissen Druck öffnet.

Der Dom (136) ragt in einen Hohlraum im hinteren Teil des Grundkörpers (101), der zur Aufnahme einer Gaspatrone (142) geeignet ist. Der Dorn (136) dient dazu, eine Gaspatrone (142), deren Öffnungsseite auf den Dorn (136) gedrückt wird, zu öffnen. Am hinteren Ende des Hohlraums ist innenseitig eine Arretierungsvorrichtung (144) und außenseitig am Grundkörper (101) ein Befestigungsmittel (146) in Form eines Außengewindes angeordnet. Die Arretierungsvorrichtung (144) dient dazu, eine ungewollte Bewegung der Verschlusskappe (117) zu verhindern. Die Arretierungsvorrichtung (144) kann dazu in Arretierungshaken (148) greifen, die an der Verschlusskappe (170) angeordnet sind. Das Befestigungsmittel (146) dient dazu, die Verschlusskappe (117) mit dem Grundkörper (101) zu verbinden, dabei die Gaspatrone (142) auf den Dorn (136) zu drücken und so zu öffnen. Dazu ist an der Verschlusskappe (117) ein Befestigungsmittel (150) in Form eines Innengewindes vorgesehen.

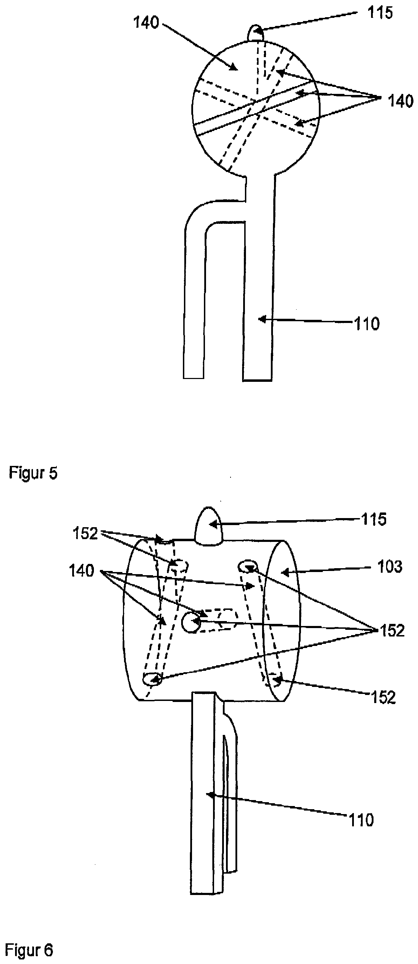

Figur 5 zeigt den Ventilkörper (103) mit Abzugsgriff (110) und Positionsanzeiger (115) nach Figur 4 in Querschnittansicht. im Inneren des Ventilkörpers (103) befinden sich drei Durchgänge (140), die sich in unterschiedlichen Winkeln zum Abzugsgriff (110) durch den Ventilkörper (103) erstrecken. Einer der drei Durchgänge (140) umfasst einen Abzweig im Ventilkörper (103), der im eingebauten Zustand zu einem Ablass führt, über den ein Entlüften der Kartuschenanschlussstücke (104, 106) und einer eingebauten Gaspatrone (142) möglich ist Figur 6 zeigt eine perspektivische Ansicht des Ventilkörpers (103) mit Abzugsgriff (110) und Positionsanzeiger (115) nach Figur 5. Auf dem Zylindermantel des Ventilkörpers (103) sind Öffnungen (152) zu erkennen, in die die drei Durchgänge (140) münden. Die Durchgänge (140) sind nebeneinander angeordnet und jeweils um circa 45° gegeneinander gekippt. Einer der Durchgänge (140) hat einen Abzweig und verbindet so drei Öffnungen (152).

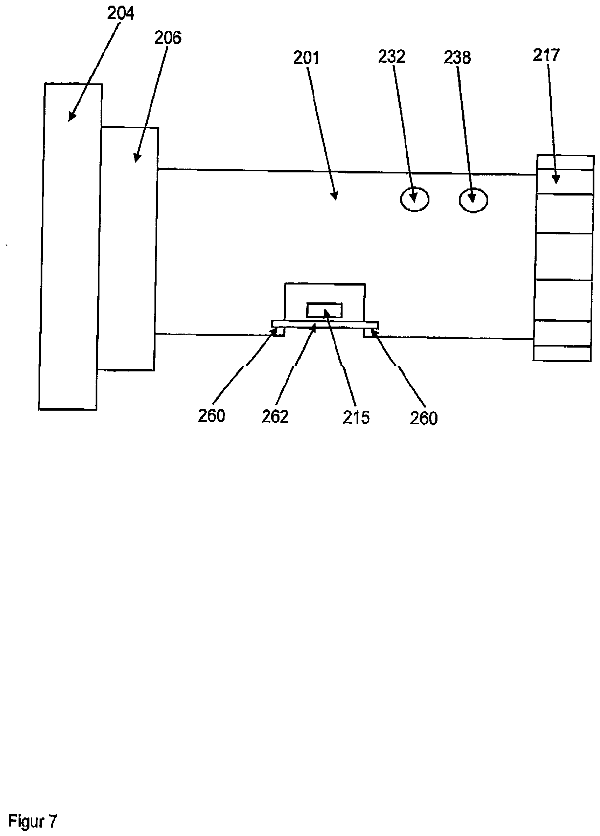

Figur 7 zeigt eine Aufsicht auf die Oberseite einer dritten erfindungsgemäßen Austragsvorrichtung umfassend einen Grundkörper (201), an dessen Vorderseite (in Figur 7 links) zwei Kartuschenanschlussstücke (204, 206) angeordnet sind, und zwar ein Kartuschenanschlussstück (204) mit großem Durchmesser für große Kartuschen und ein Kartuschenanschlussstück (206) mit kleinerem Durchmesser für kleinere Kartuschen. Die Kartuschenanschlussstücke (204, 206) umfassen eine Zylinderwand und Befestigungsmittel (nicht gezeigt) zum Befestigen der Kartuschen. Kleinere Kartuschen können durch den Innenbereich des Kartuschenanschlussstücks (204) für große Kartuschen hindurch gesteckt und in den Befestigungsmitteln des kleineren Kartuschenanschlussstücks (206) befestigt werden.

Auf der Rückseite der Austragsvorrichtung (in Figur 7 rechts) befindet sich eine Verschlusskappe (217), mit der ein Hohlraum im Grundkörper (201) zur Aufnahme einer Gaspatrone schließbar ist. Auf der Oberseite der Austragsvorrichtung sind zwei Sterilfilter (232, 238) im Bereich zweier Öffnungen angeordnet, die mit zwei Kanälen im Inneren des Grundkörpers (201) verbunden sind, wobei über die Kanäle der Hohlraum für die Gaspatrone, beziehungsweise die Gaspatrone selbst, mit den Kartuschenanschlussstücken (204, 206) verbindbar ist. im Inneren des Grundkörpers (201) befindet sich ein konischer drehbarer Ventilkörper (nicht gezeigt) in einer dafür vorgesehenen, senkrecht zur Verbindungslinie Kartuschenanschlussstück (206) und Verschlusskappe (217) angeordneten konischen Bohrung im Grundkörper (201), mit dem die verschiedenen Kanäle zwischen den Kartuschenanschlussstücken (204, 206), dem Hohlraum für die Gaspatrone, beziehungsweise die Gaspatrone, und den Öffnungen mit den Sterilfiltem (232, 238) verbindbar und verschließbar sind. Dazu können im Ventilkörper Durchgänge angeordnet sein. Ebenso können die Durchgänge aber auch durch die Außenwand des Ventilkörpers und die Innenwand des Grundkörpers (201) in dem Bereich gebildet sein, in denen der Grundkörper (201) und der Ventilkörper miteinander in Kontakt stehen. Hierzu können Furchen in den Grenzbereichen zwischen dem Grundkörper (201) und dem Ventilkörper in deren Oberflächen vorgesehen sein. Auch eine Kombination von Furchen und Durchgängen im Inneren des Ventilkörpers kommen als Durchgänge des Ventils in Betracht.

Auf der Oberseite des Ventilkörpers befindet sich ein Positionsanzeiger (215) über den die Stellung des Ventilkörpers ablesbar ist und damit welcher der Kanäle geöffnet ist, oder ob das Ventil geschlossen ist. In der Oberseite des Grundkörpers ist eine Ausnehmung (260) senkrecht zur konischen Bohrung, die zur Aufnahme des Ventilkörpers dient, angebracht, die einen Keil (262) aufnehmen kann. Die Ausnehmung (260) ist so angeordnet, dass bei Einpressen des Keils (262) der konische Ventilkörper in die konische Bohrung gepresst wird.

Der Ventilkörper ist konisch von einer Seite zur anderen verjüngt und an der größeren Seite durch den in den Grundkörper (201) in der Ausnehmung (260) eingepressten Keil (262) fixiert. Dadurch kann ein solcher Anpressdruck des Ventilkörpers in der Bohrung erreicht werden, dass die angestrebte Dichtungsfunktion gewährleistet wird. Der Keil (262) ist selbst durch in der Kunststofftechnik übliche Schnapp- oder Rastvorrichtungen gegen unerwünschte Bewegungen in Richtung der Keilachse gesichert. Alternativ ist es auch möglich, den Ventilkörper mit mindestens einem Außengewinde zu versehen und durch ein an mindestens einer Außenseite des Grundkörpers angeordnete Mutter durch Verschraubung anzupressen und zu sichern.

Auf der Rückseite erfindungsgemäßer Austragsvorrichtungen (in den Figur 1, 4 und 7 rechts) kann sich hinter dem drehbaren Ventilkörper (3, 103) im Grundkörper (1, 101, 201) ein Hohlraum für eine Gaspatrone befinden. Der Grundkörper (1, 101, 201) ist zum Einsetzen oder Herausnehmen der Gaspatrone zur Rückseite der Austragsvorrichtung hin offen. Diese Öffnung kann durch eine Verschlusskappe (17, 117, 217) abdeckbar sein. Die Verschlusskappe (17, 117, 217) kann dazu die Form eines einseitig geschlossenen Hohlzylinders haben und kann mit Befestigungsmitteln am Grundkörper (1, 101, 201) befestigt werden.

Erfindungsgemäß kann es auch sein, dass ein Grundkörper (1, 101, 201) vorhanden ist, in dem mindestens eine Bohrung (2) angebracht ist, dass eine Seite des Grundkörpers (1, 101, 201) als Kartuschenanschlussstück (4, 104, 204, 6, 106, 206) mit mindestens zwei in konzentrischen Kreisen angeordneten Fixierungsvorrichtungen (20, 120, 21, 121) zur Fixierung von Kartuschen angeordnet ist, die Gewinde oder Gewindeausschnitte bilden, dass eine andere Seite des Grundkörpers (1, 109, 201) als Pistolengriff (8,108) ausgebildet ist, dass in der Bohrung (2) ein Ventilkörper (3, 103) um seine Zylinderachse drehbar angeordnet ist, dass der Ventilkörper (3, 103) ein Ventil bildet, das a) aus dem Ventilkörper (3, 103) mit mindestens zwei Durchgängen (140) b) einem Positionsanzeiger (15, 115, 215) und (c) einem Abzugsgriff (10, 110) aufgebaut ist, wobei der Ventilkörper (3, 103) mit dem Positionsanzeiger (15, 115, 215) und dem Abzugsgriff (10, 110) verbunden ist, dass die Durchgänge (140) im Ventilkörper (3, 103) nicht auf einer Ebene angeordnet sind, dass zumindest zwei vordere Kanäle (130) im Grundkörper (1, 101, 201) angeordnet sind, welche zumindest zwei gasdurchlässige Durchlässe zwischen der Außenseite vom Kartuschenanschlussstück (4, 104, 204, 6, 106, 206) und den Durchgängen (140) bilden, die vorderen Kanäle (130) münden an der Außenseite der Kartuschenanschlussstücke (4, 104, 204, 6, 106, 206) in den Öffnungen (25, 125), die sich innerhalb der in konzentrischen Kreisen angeordneten Fixierungsvorrichtungen (20, 120, 21, 121) zur Fixierung von Kartuschen befinden, dass zumindest ein Ablasskanal (131) mit einem der Durchgänge (140) des Ventils mit einem drucksensitiven Sicherheitsventil verbindbar ist, das bei zuvor eingestelltem Gasdruck, eine Verbindung zwischen einem vorderen Kanal (130) und einem hinteren Kanal (135) und der äußeren Umgebung herstellt, dass zwischen dem Sicherheitsventil und der Umgebung ein Sterilfilter (132) angeordnet ist, dass mindestens ein Durchgang (140) des Ventilkörpers (3, 103) mit einem hinteren Kanal (135) kommunizieren kann, der mit der Gaspatrone (142) verbunden ist, dass der hintere Kanal (135) mit mindestens einem Durchgang (140) des Ventilkörpers (3,103) und einem vorderen Kanal (130) kommunizieren kann, dass ein zweiter hinterer Kanal (135) mit einem daran angeschlossenen Ablasskanal (137) eine Verbindung zwischen mindestens einem Durchgang (140) im Ventilkörper (3, 103) und der Umgebung herstellt, dass zwischen dem Ablasskanal (137) und der Umgebung ein Sterilfilter (138) angeordnet ist, dass der zweite hintere Kanal (135) über mindestens einen Durchgang (140) im Ventilkörper (3, 103) mit einem zweiten vorderen Kanal (130) gasdurchlässig verbunden werden kann.

Eine vorteilhafte Ausgestaltung der Erfindung kann zusätzlich darin bestehen, dass die Gasentnahmeöffnung der Gaspatrone (142) durch einen einsteckbaren, herausziehbaren Stift oder durch eine herausdrehbare Platte so gesichert ist, dass die Gaspatrone (142) vor der Applikation nicht durch Einwirkung von mechanischen Kräften, wie sie beim Transport auftreten können, aufgestochen wird. Daneben ist es auch möglich, den Ventilkörper (3, 103) durch einen herausziehbaren Stift oder eine Platte gegen die unbeabsichtigte Verdrehung zu schützen. Besonders vorteilhaft ist es, wenn die Sicherung der Gaspatrone (142) und des Ventilkörpers (3, 103) eine Einheit bilden und gemeinsam mit möglichst einen Handgriff vor der Applikation entfernt oder inaktiviert werden können. Die Entsicherung der Austragsvorrichtung kann zum Beispiel mit dem Öffnen der Gaspatrone (142) gekoppelt sein.

Der Grundkörper (1, 101, 201) der Austragsvorrichtung bildet bevorzugt mit dem Kartuschenanschlussstück mit zwei in Kreisen angeordneten Fixierungsvorrichtungen (20, 21, 124, 121) zur Fixierung von Kartuschen und dem Pistolengriff (8, 108) eine Einheit. Diese Teile sind als zusammenhängende Einheit bevorzugt als ein Spritzgießteil ausgebildet.

Die Anordnung von zwei Fixierungsvorrichtungen (20, 21, 120, 121) zur Fixierung von Kartuschen in zwei konzentrischen Ringen hat den Vorteil, dass mit dem inneren Ring Kartuschen mit einem entsprechenden kleinen Durchmesser, wie er bei einzelnen Kartuschen üblich ist, fixiert werden können und dass bei Verwendung von Kartuschen mit größerem Durchmesser, zum Beispiel bei Mehrkomponentenkartuschen, auch diese mit der derselben Austragsvorrichtung verbunden werden können. Der Vorteil besteht darin, dass mit einer solchen Austragsvorrichtung Kartuschen unterschiedlicher Durchmesser ausgepresst werden können. Dadurch kann die Wirtschaftlichkeit der Austragsvorrichtung verbessert werden.

Neben der Anordnung von Fixierungsvorrichtungen (20, 21, 120, 121) zur Fixierung von Kartuschen in zwei konzentrischen Ringen ist es auch im Rahmen der Erfindung, dass die Fixierungsvorrichtungen (20, 21, 120, 121) auch in nicht zueinander konzentrischen Ringen oder auch in nicht kreisförmigen Anordnungen ausgebildet sein können.

Das Ventil ist durch einen mit mindestens zwei Durchgängen durchbohrten Ventilkörper (3, 103) gebildet, an den ein Positionsanzeiger (15, 115, 215) und ein Abzugsgriffstück (10, 110) angebracht sind. Das gesamte Ventil einschließlich des Positionsanzeigers (15, 115, 215) und des Abzugsgriffstücks (10, 110) ist bevorzugt einteilig und kann als Einzelteil durch Spritzgießen gefertigt werden. Bei Ziehen des Abzugsgriffstücks (10, 110) in Richtung Pistolengriff (8, 108) wird der Ventilkörper (3, 103) so gedreht, dass die vorderen Kanäle (130) und die hinteren Kanäle (135) durch die Durchgänge (140) gasdurchlässig verbunden sind. Bei zuvor geöffneter Gaspatrone erfolgt dann ein Gasfluss in Richtung der Kartuschenanschlussstücke (4, 104, 204, 6, 106, 206). Das Gas strömt aus der Öffnung (25, 125) und drückt auf den Kolben der zuvor eingesetzten Kartusche. Dadurch wird der Kolben in Richtung Kartuschenkopf bewegt und presst dadurch das fließfähige Material aus der Kartusche.

Wenn das Abzugsgriffstück (10, 110) in Richtung des Kartuschenanschlussstücks (4, 104, 204, 6, 106,206) bewegt wird, so dreht sich der Ventilkörper (3, 103) so, dass der Durchgang (140) nicht mehr die durchgehenden vorderen und hinteren Kanäle (130, 135) verbindet. Bei senkrechter Stellung des Abzugsgriffstücks (10, 110) gibt es keine Verbindung der Kanäle (130, 135) miteinander. Wenn das Abzugsgriffstück (10, 110) weiter in Richtung Kartuschenanschlussstück (4, 104, 204, 6, 106, 206) gedreht wird, verbindet ein Durchgang (140) einen durchgehenden vorderen Kanal (130) und den mit dem ersten Ablasskanal (137) verbundenen hinteren Kanal (135) miteinander. Das komprimierte Gas strömt aus dem Kartuschenanschlussstück (4, 104, 204, 6, 106, 206) durch eine der Öffnungen (125) durch einen der durchgehenden vorderen Kanäle (130), durch einen der Durchgänge (140) und durch den ersten Ablasskanal (137) zum Sterilfilter (138). Das Gas tritt durch den Sterilfilter (138) in die Umgebung aus. Dadurch kann komprimiertes Gas aus dem Raum zwischen dem Kartuschenkolben und dem Kartuschenanschlussstück (4, 104, 204, 6, 106, 206) über den Sterilfilter (138) in die Umgebung abgegeben werden. Die Bewegung des Kolbens wird dadurch sofort gestoppt.

Der an der Oberseite der Austragsvorrichtung herausragende Positionsanzeiger (15, 115, 215) wird automatisch bei Betätigung des Abzuggriffstücks (10, 110) mit bewegt, weil der Positionsanzeiger (15, 115, 215) auf der Oberseite des Ventilkörpers (3, 103) angeordnet ist. Das bedeutet, der Positionsanzeiger (15, 115, 215) zeigt den Verschlusszustand des Ventils an. Der Anwender erhält somit jederzeit bei Gebrauch der Austragsvorrichtung die Information über den Verschlusszustand des Ventils.

Es ist erfindungsgemäß, dass die Durchgänge (140) im Ventilkörper (3, 103) um einen Winkel von mindestens 10° versetzt in einer Ebene senkrecht zur Achse des Zylinders angeordnet sind.

Weiterhin kann erfindungsgemäß vorgesehen sein, dass im Grundkörper (1, 101, 201) vordere Kanäle (130) und hintere Kanäle (135) angeordnet sind, wobei der erste vordere Kanal (130) die Außenseite des Kartuschenanschlussstücks (4, 104, 204, 6, 106, 206) mit einem ersten Durchgang (140) im Ventilkörper (3, 103) verbindet, wobei der erste hintere Kanal (135) den ersten Durchgang (140) mit einem Sterilfilter (138) verbindet, der mit der Umgebungsatmosphäre verbunden ist und das der Ventilkörper (3, 103) einen zweiten Durchgang (140) umfasst, welche einen zweiten vorderen Kanal (130) und einen zweiten hinteren Kanal (135) so miteinander verbindet, dass eine Gasdurchströmung gewährleistet ist.

Das Abzugsgriffstück (10, 110) ist bevorzugt U-förmig ausgebildet. Dadurch ist es möglich, mit einer einfachen Bewegung mit der Hand das Griffstück in Richtung Kartuschenanschlussstück zu drücken und damit den Gasfluss und damit den Fluss des pastösen Materials zu stoppen. Bei weiterer Bewegung des Abzugsgriffstücks (10, 110) in Richtung Kartuschenanschlussstück (4, 104, 204, 6, 106, 206) kann das komprimierte Gas über eine erste Öffnung (25, 125) durch den ersten vorderen Kanal (130), durch den ersten Durchgang (140), durch den ersten hinteren Kanal (135), durch den Ablasskanal (137) und den Sterilfilter (138) in die Umgebung abgelassen werden.

Die Sterilfilter (132, 232, 138, 238) können aus medizinischem Papier, gasdurchlässigen Kunststoffmembranen, gasdurchlässigen Kunststoffporenscheiben, gasdurchlässigen Metallporenscheiben, gasdurchlässigen Glasporenscheiben oder aus gasdurchlässigen Kunststoffvliesen gefertigt sein.

Es kann auch erfindungsgemäß vorgesehen sein, dass die Bohrung (2) im Grundkörper (1, 101, 201) bevorzugt konisch von einer Außenseite zur anderen Außenseite verjüngt ist und dass im Grundkörper (1, 101, 201) auf der Seite der größeren Öffnung der Bohrung (2) eine Ausnehmung (260) senkrecht zur Bohrung (2) vorgesehen ist, die einen Keil (262) aufnehmen kann. Die Ausnehmung (260) ist so angeordnet, dass bei Einpressen des Keils (262) der Ventilkörper (3,103) in die Bohrung (2) gepresst wird.

Spezielle Ausgestaltungen der vorliegenden Erfindung können auch dadurch gekennzeichnet sein, dass der Ventilkörper (3, 103) bevorzugt konisch von einer Seite zur anderen Seite verjüngt ist und dass der Ventilkörper (3, 103) an der größeren Seite durch einen in den Grundkörper (1, 101, 201) in die Ausnehmung (260) eingepressten Keil (262) fixiert ist. Dadurch kann ein solcher Anpressdruck des Ventilkörpers (3, 103) in der Bohrung (2) erreicht werden, dass die angestrebte Dichtungsfunktion gewährleistet wird. Der Keil (262) kann selbst durch in der Kunststofftechnik übliche Schnapp- oder Rastvorrichtungen gegen unerwünschte Bewegungen in Richtung der Keilachse gesichert sein. Alternativ ist es auch möglich, den Ventilkörper (3, 103) mit mindestens einem Außengewinde an den Zylinderende zu versehen und durch ein an mindestens einer Außenseite des Grundkörpers (1, 101, 201) angeordnete Mutter durch Verschraubung anzupressen und zu sichern.

Erfindungsgemäße Austragsvorrichtungen sind bevorzugt im Wesentlichen aus medizinisch akzeptablen Kunstoffen gefertigt.

Erfindungsgemäße Austragsvorrichtungen sind besonders bevorzugt zum Auspressen von fließfähigen Massen aus Kartuschen geeignet, wobei die fließfähigen Massen direkt in den Kartuschen gelagert sind, und zum Auspressen von fließfähigen Massen, die in Beuteln gelagert sind, die sich wiederum in Kartuschen befinden, geeignet.

Erfindungsgemäße Austragsvorrichtungen werden zum Austragen von Knochenzement aus Kartuschen verwendet.

Die erfindungsgemäße Austragsvorrichtung kann zum einmaligen und mehrmaligen Gebrauch, bestimmt sein. Aufgrund der geringen Herstellungskosten ist der einmalige Gebrauch besonders geeignet.

### Bezugszeichenliste

- 1, 101, 201: Grundkörper
- 2: Öffnung
- 3, 103: Ventilkörper
- 4, 104, 204: Kartuschenanschlussstück mit großem Durchmesser
- 6, 106, 206: Kartuschenanschlussstück mit kleinem Durchmesser
- 8, 108: Pistolengriff
- 10, 110: Abzugsgriff
- 15, 915, 215: Positionsanzeiger
- 17, 117, 217: Verschlusskappe
- 20, 120: Fixierungsvorrichtung
- 21, 121: Fixierungsvorrichtung
- 25, 125: Öffnung im Kartuschenanschlussstück
- 26: Rastvorrichtung
- 130: vorderer Kanal
- 131, 137: Ablasskanal
- 132, 232: Sterilfilter
- 135: hinterer Kanal
- 136: hohler Dorn
- 138, 238: Sterilfilter
- 140: Durchgang
- 142: Gaspatrone
- 144: Arretierungsvorrichtung
- 146: Befestigungsmittel
- 148: Arretierungshaken
- 150: Befestigungsmittel
- 152: Öffnungen im Ventilkörper
- 260: Ausnehmung
- 262: Keil

## Patentansprüche

1. Austragsvorrichtung für das Austragen von Knochenzement aus Kartuschen umfassend einen Anschluss (136) zum Einspeisen eines unter Druck stehenden Gases, einen Grundkörper (1, 101, 201), in dem wenigstens ein Durchgang (140) und/oder Kanal (130, 135) verläuft, durch den das unter Druck stehende Gas zu einem ersten Kartuschenanschlussstück (6, 106, 206) zum Anschließen einer Kartusche leitbar ist, wobei das erste Kartuschenanschlussstück (6, 106, 206) eine erste Öffnung (25, 125) zu dem Durchgang (140) und/oder dem vorderen Kanal (130) umfasst, so dass bei Beaufschlagung einer im Kartuschenanschlussstück (6, 106, 206) angeordneten Kartusche mit dem unter Druck stehenden Gas, ein Kartuscheninhalt aus der Kartusche austreibbar ist, und
**dadurch gekennzeichnet, dass**
am vorderen Rand des ersten Kartuschenanschlussstücks (6, 106, 206) ein zweites Kartuschenanschlussstück (4, 104,204) mit einem größeren Querschnitt und einer zweiten Öffnung angeordnet ist, wobei das zweite Kartuschenanschlussstück (4, 104, 204) zum Anschließen einer größeren Kartusche geeignet ist, und wobei die zweite Öffnung des zweiten Kartuschenanschlussstücks (4, 104, 204) so groß ist, dass eine Kartusche durch die zweite Öffnung hindurch am ersten Kartuschenanschlussstück (6, 106, 206) anschließbar ist.

2. Austragsvorrichtung nach Anspruch 1, **dadurch gekennzeichnet, dass**
die Kartuschenanschlussstücke (4, 104, 204, 6, 106, 206) zylindrische Hohlkörper mit einer geschlossenen Seite sind, wobei die geschlossene Seite des zweiten Kartuschenanschlussstücks (4, 104,204) die zweite Öffnung umfasst und die geschlossene Seite des ersten Kartuschenanschlussstücks (6, 106, 206) die erste Öffnung (25, 125) umfasst.

3. Austragsvorrichtung nach Anspruch 1 oder 2, **dadurch gekennzeichnet, dass**
auf der Innenseite der Kartuschenanschlussstücke (4, 104, 204,6, 106, 206) Fixierungsvorrichtungen (20, 120, 21, 121) zum Fixieren von Kartuschen angeordnet sind.

4. Austragsvorrichtung nach Anspruch 3, **dadurch gekennzeichnet, dass**
die Fixierungsvorrichtungen (20, 120, 21, 121) in zwei konzentrischen Kreisen an den Kartuschenanschlussstücken (4, 104, 204, 6, 106, 206) angeordnet sind.

5. Austragsvorrichtung nach Anspruch 3 oder 4, **dadurch gekennzeichnet, dass**
die Fixierungsvorrichtungen (20, 120, 21, 121) Gewinde, insbesondere Innengewinde, sind.

6. Austragsvorrichtung nach Anspruch 3 oder 4, **dadurch gekennzeichnet, dass**
die Fixierungsvorrichtungen (20, 120, 21, 121) Rastvorrichtungen (26), insbesondere in Form von in eine Richtung deformierbarer Zapfen umfassen.

7. Austragsvorrichtung nach einem der vorangehenden Ansprüche, **dadurch gekennzeichnet, dass**
die Austragsvorrichtung einen Abzugsgriff (10, 110) zum Bedienen eines Ventils der Austragsvorrichtung und einen Grundkörper (1, 101, 201) umfasst, in dem wenigstens ein durch das bedienbare Ventil schließbarer und zu öffnender Durchgang (140) verläuft, durch den das unter Druck stehende Gas zu den Kartuschenanschlussstücken (4, 104, 204, 6, 106, 206) leitbar ist, wobei die Öffnung (25, 125) zu dem Durchgang (140) führt, wobei das Ventil ein Ventilkörper (3, 103) ist, der drehbar im Grundkörper (1, 101, 201) angeordnet ist, wobei eine Bewegung des Abzugsgriffs (10, 110) zu einer Drehung des Ventilkörpers (3, 103) im Grundkörper (1, 101, 201) führt und der Durchgang (140) im oder am Ventilkörper (3, 103) angeordnet ist, so dass in einer ersten Position des Ventilkörpers (3, 103) der Durchgang (140) geschlossen ist und in einer zweiten Position des Ventilkörpers (3, 103) der Durchgang (140) im oder am Ventilkörper (3, 103) das unter Druck stehende Gas zu den Kartuschenanschlussstücken (4, 104, 204, 6, 106, 206) leitet.

8. Austragsvorrichtung nach Anspruch 7, **dadurch gekennzeichnet, dass**
zwischen zumindest einem Durchgang (140) und dem Kartuschenanschlussstück (6, 106, 206) zumindest ein vorderer Kanal (130) im Grundkörper (1, 101, 201) angeordnet ist und/oder zwischen zumindest einem Durchgang (140) und dem Anschluss (136) zum Einspeisen des unter Druck stehenden Gases zumindest ein hinterer Kanal (135) angeordnet ist.

9. Austragsvorrichtung nach einem der Ansprüche 7 oder 8, **dadurch gekennzeichnet, dass**
ein zweiter Durchgang (140) im oder am Ventilkörper (3, 103) angeordnet ist, der in einer dritten Position des Ventilkörpers (3, 103) die Kartuschenanschlussstücke (4, 104, 204, 6, 106, 206) mit einem ersten Ablass verbindet, so dass ein Überdruck an den Kartuschenanschlusstücken (4, 104, 204, 6, 106, 206) in der dritten Position des Ventilkörpers (3, 103) durch den ersten Ablass entweichen kann.

10. Austragsvorrichtung nach Anspruch 9, **dadurch gekennzeichnet, dass**
ein erster Ablasskanal (137) im Grundkörper (1, 101, 201) derart angeordnet ist, dass er in der dritten Position des Ventilkörpers (3, 103) den zweiten Durchgang (140) mit dem ersten Ablass verbindet.

11. Austragsvorrichtung nach einem der Ansprüche 7 bis 10, **dadurch gekennzeichnet, dass**
ein dritter Durchgang (140) im oder am Ventilkörper (3, 103) angeordnet ist, der einen Abzweig umfasst und der in einer vierten Position des Ventilkörpers (3, 103) die Kartuschenanschlussstücke (4, 104, 204, 6, 106, 206) und das unter Druck stehende Gas mit einem zweiten Ablass verbindet, so dass ein Überdruck in der Austragsvorrichtung in der vierten Position des Ventilkörpers (3, 103) durch den zweiten Ablass entweichen kann.

12. Austragsvorrichtung nach Anspruch 11, **dadurch gekennzeichnet, dass**
zwischen dem dritten Durchgang (140) und dem Anschluss (136) zum Einspeisen des unter Druck stehenden Gases ein zweiter hinterer Kanal (135) im Grundkörper (1, 101, 201) angeordnet ist und/oder ein zweiter Ablasskanal (131) im Grundkörper (1, 101, 201) derart angeordnet ist, dass er in der vierten Position des Ventilkörpers (3, 103) den Abzweig des dritten Durchgangs (140) mit dem zweiten Ablass verbindet.

13. Austragsvorrichtung nach einem der Ansprüche 9 bis 10, **dadurch gekennzeichnet, dass**
an zumindest einem der Ablässe ein Sterilfilter (132, 138) angeordnet ist und/oder in zumindest einem der Ablässe ein Überdruckventil angeordnet ist.

14. Austragsvorrichtung nach einem der Ansprüche 7 bis 13, **dadurch gekennzeichnet, dass**
der Abzugsgriff (10, 110) fest an der Unterseite des drehbaren Ventilkörpers (3, 103) angeordnet ist, so dass mit dem Abzugsgriff (10, 110) der Ventilkörper (3, 103) im Grundkörper (1, 101, 201) drehbar ist.

15. Austragsvorrichtung nach einem der Ansprüche 7 bis 14, **dadurch gekennzeichnet, dass** am drehbaren Ventilkörper (3, 103) ein Positionsanzeiger (15, 115, 215), insbesondere an der Oberseite, angeordnet ist, an dem die Position des Ventilkörpers (3, 103) im Grundkörper (1, 101, 201) ablesbar ist.

16. Austragsvorrichtung nach einem der vorangehenden Ansprüche, **dadurch gekennzeichnet, dass**
an der Unterseite der Austragsvorrichtung ein Griff (8, 108) zum Halten der Austragsvorrichtung angeordnet ist.

17. Austragsvorrichtung nach einem der Ansprüche 7 bis 16, **dadurch gekennzeichnet, dass** der Ventilkörper (3, 103) ein konisch geformter, zumindest bereichsweise rotationssymmetrischer Körper ist, der in einer zur Form des Ventilkörpers (3, 103) passenden konischen Öffnung (2) im Grundkörper (1, 101,201) drehbar gelagert ist, wobei die Außenwände des Ventilkörpers (3, 103) bis auf die Öffnungen der Durchgänge (140) dicht mit den Wänden der konischen Öffnung (2) im Grundkörper (1, 101, 201) in jeder Position des Ventilkörpers (3, 103) abschließen, so dass die Verbindungen der Durchgänge (140) zu zumindest einem Kanal (130, 131, 135, 137) im Grundkörper (1, 101, 201) dicht sind.

18. Austragsvorrichtung nach Anspruch 17, **dadurch gekennzeichnet, dass**
im Grundkörper (1, 101, 201) eine Ausnehmung (260) für einen Keil (262) vorgesehen ist, wobei ein in die Ausnehmung (260) eingeschobener Keil (262) den Ventilkörper (3, 103) in die konische Öffnung (2) im Grundkörper (1, 101, 201) presst, so dass der Ventilkörper (3, 103) einen gasdichten Presssitz in der konischen Öffnung (2) erzeugt.

19. Austragsvorrichtung nach einem der vorangehenden Ansprüche, **dadurch gekennzeichnet, dass**
an der den Kartuschenanschlussstücken (4, 104, 204, 6, 106, 206) abgewandten Seite der Austragsvorrichtung, insbesondere im Grundkörper (1, 101, 201), ein Hohlraum für eine Gaspatrone vorgesehen ist, wobei der Anschluss (136) zum Einspeisen eines unter Druck stehenden Gases in dem Hohlraum angeordnet ist und einen Dorn (136) zum Öffnen einer Gaspatrone umfasst, und am Grundkörper (1, 101, 201) Befestigungsmittel (146) zum Befestigen einer Verschlusskappe (17, 117, 217) mit Befestigungsmitteln (150) angeordnet sind, wobei die Gaspatrone (142) durch das Befestigen der Verschlusskappe (17, 117, 217) zu öffnen ist, wobei vorzugsweise die Verschlusskappe (17, 117, 217) zusätzlich ein Arretierungsmittel (148) umfasst, das in eine Arretierungsvorrichtung (144) im Hohlraum greift und wobei besonders bevorzugt die Arretierungsvorrichtung (144) eine eingesetzte Gaspatrone (142) geeigneter Größe im Hohlraum positioniert.

20. Austragsvorrichtung nach einem der Ansprüche 9 bis 19, **dadurch gekennzeichnet, dass** die Durchgänge (140) im Wesentlichen gerade durch das Innere des Ventilkörpers (3, 103) verlaufen und die Durchgänge (140) um einen Winkel von 10 ° bis 80 ° um die Drehachse des Ventilkörpers (3, 103) gegeneinander versetzt sind.

## Claims

1. A dispensing device for dispensing bone cement from cartridges, comprising a connector (136) for feeding a pressurised gas, a base body (1, 101, 201), in which at least one passage (140) and/or channel (130, 135) runs, through which the pressurised gas can be guided to a first cartridge connecting piece (6, 106, 206) for connecting a cartridge, wherein the first cartridge connecting piece (6, 106, 206) comprises a first opening (25, 125) to the passage (140) and/or to the front channel (130), so that a cartridge content can be expelled from the cartridge upon applying the pressurised gas to a cartridge arranged in the cartridge connecting piece (6, 106, 206), and
**characterised in that**
a second cartridge connecting piece (4, 104, 204) comprising a larger cross section and a second opening is arranged on the front edge of the first cartridge connecting piece (6, 106, 206), wherein the second cartridge connecting piece (4, 104, 204) is suitable for connecting a larger cartridge, and wherein the second opening of the second cartridge connecting piece (4, 104, 204) is so large that a cartridge can be connected to the first cartridge connecting piece (6, 106, 206) through the second opening.

2. The dispensing device according to claim 1, **characterised in that** the cartridge connecting pieces (4, 104, 204, 6, 106, 206) are cylindrical hollow bodies comprising a closed side, wherein the closed side of the second cartridge connecting piece (4, 104, 204) encompasses the second opening, and the closed side of the first cartridge connecting piece (6, 106, 206) encompasses the first opening (25, 125).

3. The dispensing device according to claim 1 or 2, **characterised in that** securing devices (20, 120, 21, 121) for securing cartridges are arranged on the inner side of the cartridge connecting pieces (4, 104, 204, 6, 106, 206).

4. The dispensing device according to claim 3, **characterised in that** the securing devices (20, 120, 21, 121) are arranged in two concentric circles on the cartridge connecting pieces (4, 104, 204, 6, 106, 206).

5. The dispensing device according to claim 3 or 4, **characterised in that** the securing devices (20, 120, 21, 121) are threads, in particular internal threads.

6. The dispensing device according to claim 3 or 4, **characterised in that** the securing devices (20, 120, 21, 121) comprise locking devices (26), in particular in the form of pins, which can be deformed in one direction.

7. The dispensing device according to any one of the preceding claims,
**characterised in that**
the dispensing device has a trigger grip (10, 110) for operating a valve of the dispensing device and a base body (1, 101, 201), in which at least one passage (140) runs, which can be closed and opened by the operable valve and through which the pressurised gas can be guided to the cartridge connecting pieces (4, 104, 204, 6, 106, 206), wherein the opening (25, 125) leads to the passage (140), wherein the valve is a valve body (3, 103), which is rotatably arranged in the base body (1, 101, 201), wherein a movement of the trigger grip (10, 110) leads to a rotation of the valve body (3, 103) in the base body (1, 101, 201), and the passage (140) is arranged in or on the valve body (3, 103), so that the passage (140) is closed in a first position of the valve body (3, 103) and the passage (140) in or on the valve body (3, 103) guides the pressurised gas to the cartridge connecting pieces (4, 104, 204, 6, 106, 206) in a second position of the valve body (3, 103).

8. The dispensing device according to claim 7, **characterised in that** at least one front channel (130) is arranged in the base body (1, 101, 201) at least between a passage (140) and the cartridge connecting piece (6, 106, 206), and/or at least one rear channel (135) is arranged between at least one passage (140) and the connector (136) for feeding the pressurised gas.

9. The dispensing device according to any one of claims 7 or 8, **characterised in that** a second passage (140) is arranged in or on the valve body (3, 103), which connects the cartridge connecting pieces (4, 104, 204, 6, 106, 206) to a first discharge in a third position of the valve body (3, 103), so that an overpressure at the cartridge connecting pieces (4, 104, 204, 6, 106, 206) in the third position of the valve body (3, 103) can escape through the first discharge.

10. The dispensing device according to claim 9, **characterised in that** a first discharge channel (137) is arranged in the base body (1, 101, 201) in such a way that it connects the second passage (140) to the first discharge in the third position of the valve body (3, 103).

11. The dispensing device according to any one of claims 7 to 10, **characterised in that** a third passage (140) is arranged in or on the valve body (3, 103), which comprises a branch, and connects the cartridge connecting pieces (4, 104, 204, 6, 106, 206) and the pressurised gas to a second discharge in a fourth position of the valve body (3, 103), so that an overpressure in the dispensing device can escape through the second discharge in the fourth position of the valve body (3, 103).

12. The dispensing device according to claim 11, **characterised in that** a second rear channel (135) is arranged in the base body (1, 101, 201) between the third passage (140) and the connector (136) for feeding in the pressurised gas and/or a second discharge channel (131) is arranged in the base body (1, 101, 201) in such a way that it connects the branch of the third passage (140) to the second discharge in the fourth position of the valve body (3, 103).

13. The dispensing device according to any one of claims 9 to 10, **characterised in that** a sterile filter (132, 138) is arranged on at least one of the discharges and/or a pressure relief valve is arranged in at least one of the discharges.

14. The dispensing device according to any one of claims 7 to 13, **characterised in that** the trigger grip (10, 110) is arranged firmly on the underside of the rotatable valve body (3, 103), so that the valve body (3, 103) can be rotated in the base body (1, 101, 201) by means of the trigger grip (10, 110).

15. The dispensing device according to any one of claims 7 to 14, **characterised in that** a position indicator (15, 115, 215) is arranged on the rotatable valve body (3, 103), in particular on the top side, on which the position of the valve body (3, 103) in the base body (1, 101, 201) can be read.

16. The dispensing device according to any one of the preceding claims,
**characterised in that**
a handle (8, 108) for holding the dispensing device is arranged on the underside of the dispensing device.

17. The dispensing device according to any one of claims 7 to 16, **characterised in that** the valve body (3, 103) is a conically shaped body, which is rotationally symmetrical at least in some areas, and is mounted in rotatable manner in a conical opening (2) in the base body (1, 101, 201), which matches the shape of the valve body (3, 103), wherein the external walls of the valve body (3, 103), except for the openings of the passages (140), end in a sealed manner against the walls of the conical opening (2) in the base body (1, 101, 201) in each position of the valve body (3, 103), so that the connections of the passages (140) to at least one channel (130, 131, 135, 137) in the base body (1, 101, 201) are sealed.

18. The dispensing device according to claim 17, **characterised in that** a recess (260) for a wedge (262) is provided in the base body (1, 101, 201), wherein a wedge (262), which is inserted into the recess (260), presses the valve body (3, 103) into the conical opening (2) in the base body (1, 101, 201), so that the valve body (3, 103) generates a gas-tight press-fit in the conical opening (2).

19. The dispensing device according to any one of the preceding claims,
**characterised in that**
a hollow space for a gas cartridge is provided on the side of the dispensing device facing away from the cartridge connecting pieces (4, 104, 204, 6, 106, 206), in particular in the base body (1, 101, 201), wherein the connector (136) is arranged in the hollow space for feeding a pressurised gas and comprises a mandrel (136) for opening a gas cartridge, and fastening means (146) for fastening a closure cap (17, 117, 217) with fastening means (150) are arranged on the base body (1, 101, 201), wherein the gas cartridge (142) is to be opened by the fastening of the closure cap (17, 117, 217), wherein the closure cap (17, 117, 217) preferably additionally comprises a locking means (148), which engages with a locking device (144) in the hollow space, and wherein the locking device (144) particularly preferably positions an inserted gas cartridge (142) of appropriate size in the hollow space.

20. The dispensing device according to any one of claims 9 to 19, **characterised in that** the passages (140) run essentially straight through the interior of the valve body (3, 103), and the passage (140) are offset with respect to one another by an angle of between 10° and 80° about the axis of rotation of the valve body (3, 103).

## Revendications

1. Dispositif d'évacuation pour le déversement de ciment osseux provenant de cartouches, comprenant une prise (136) pour l'alimentation d'un gaz sous pression, un corps de base (1, 101, 201), dans lequel passe au moins un passage (140) et/ou conduit (130, 135), par lequel le gaz sous pression peut être acheminé vers une première pièce de jonction de la cartouche (6, 106, 206) pour le raccordement d'une cartouche, la première pièce de jonction de la cartouche (6, 106, 206) comprenant un premier orifice (25, 125) vers le passage (140) et/ou le conduit avant (130) de façon à ce que, lors de l'alimentation d'une cartouche contenant le gaz sous pression, disposée dans la pièce de jonction de la cartouche (6, 106, 206), un contenu de la cartouche puisse être expulsé de la cartouche et
**caractérisé en ce que**
une deuxième pièce de jonction de la cartouche (4, 104, 204) avec une section plus grande et un deuxième orifice est disposée sur le bord frontal de la première pièce de jonction de la cartouche (6, 106, 206), la deuxième pièce de jonction de la cartouche (4, 104, 204) étant appropriée pour le raccordement d'une plus grande cartouche et la taille du deuxième orifice de la deuxième pièce de jonction de la cartouche (4, 104, 204) permettant le raccordement d'une cartouche à la première pièce de jonction de la cartouche (6, 106, 206) par le deuxième orifice.

2. Dispositif d'évacuation conformément à la revendication n°1, **caractérisé en ce que**
les pièces de jonction de la cartouche (4, 104, 204, 6, 106, 206) sont des corps creux cylindriques avec un côté fermé, le côté fermé de la deuxième pièce de jonction de la cartouche (4, 104, 204) comprenant le deuxième orifice et le côté fermé de la première pièce de jonction de la cartouche (6, 106, 206) comprenant le premier orifice (25, 125).

3. Dispositif d'évacuation conformément à la revendication n°1 ou n°2,
**caractérisé en ce que**
des dispositifs de fixation (20, 120, 21, 121) pour la fixation de cartouches sont disposés sur la face intérieure des pièces de jonction de la cartouche (4, 104, 204, 6, 106, 206).

4. Dispositif d'évacuation conformément à la revendication n°3, **caractérisé en ce que**
les dispositifs de fixation (20, 120, 21, 121) sont disposés dans deux cercles concentriques sur les pièces de jonction de la cartouche (4, 104, 204, 6, 106, 206).

5. Dispositif d'évacuation conformément à la revendication n°3 ou n°4,
**caractérisé en ce que**
les dispositifs de fixation (20, 120, 21, 121) sont des filetages, notamment des filetages intérieurs.

6. Dispositif d'évacuation conformément à la revendication n°3 ou n°4,
**caractérisé en ce que**
les dispositifs de fixation (20, 120, 21, 121) sont des dispositifs d'encliquetage (26), en particulier sous forme de tenons déformables dans une direction.

7. Dispositif d'évacuation conformément à l'une des revendications précédentes,
**caractérisé en ce que**
le dispositif d'évacuation comprend une poignée de détente (10, 110) pour l'actionnement d'une vanne du dispositif d'évacuation et un corps de base (1, 101, 201) dans lequel passe au moins un passage (140) pouvant être fermé et ouvert par la vanne actionnable, par lequel le gaz sous pression peut être acheminé vers les pièces de jonction de la cartouche (4, 104, 204, 6, 106, 206), l'orifice (25, 125) menant au passage (140), la vanne étant un corps de vanne (3, 103) qui est disposé de façon rotative dans le corps de base (1, 101, 201), un mouvement de la poignée de détente (10, 110) menant à une rotation du corps de vanne (3, 103) dans le corps de base (1, 101, 201) et le passage (140) étant disposé dans ou sur le corps de vanne (3, 103) de façon à ce que le passage (140) soit fermé dans une première position du corps de vanne (3, 103) et que le passage (140) dans ou sur le corps de vanne (3, 103) achemine le gaz sous pression vers les pièces de jonction de la cartouche (4, 104, 204, 6, 106, 206) dans une deuxième position du corps de vanne (3, 103).

8. Dispositif d'évacuation conformément à la revendication n°7, **caractérisé en ce que**
au moins un conduit avant (130) est disposé dans le corps de base (1, 101, 201) entre au moins un passage (140) et la pièce de jonction de la cartouche (6, 106, 206) et/ou qu'au moins un conduit arrière (135) est disposé entre au moins un passage (140) et la prise (136) pour l'alimentation du gaz sous pression.

9. Dispositif d'évacuation conformément à l'une des revendications n°7 ou n°8,
**caractérisé en ce que**
un deuxième passage (140) est disposé dans ou sur le corps de vanne (3, 103), lequel relie, dans une troisième position du corps de vanne (3, 103), les pièces de jonction de la cartouche (4, 104, 204, 6, 106, 206) à une première évacuation de façon à ce qu'une surpression au niveau des pièces de jonction de la cartouche (4, 104, 204, 6, 106, 206) puisse s'échapper par la première évacuation dans la troisième position du corps de vanne (3, 103).

10. Dispositif d'évacuation conformément à la revendication n°9, **caractérisé en ce que**
un premier conduit d'évacuation (137) est disposé dans le corps de base (1, 101, 201) de telle manière à relier, dans la troisième position du corps de vanne (3, 103), le deuxième passage (140) à la première évacuation.

11. Dispositif d'évacuation conformément à l'une des revendications n°7 à n°10,
**caractérisé en ce que**
un troisième passage (140) est disposé dans ou sur le corps de vanne (3, 103), lequel comprend une dérivation et relie, dans une quatrième position du corps de vanne (3, 103), les pièces de jonction de la cartouche (4, 104, 204, 6, 106, 206) et le gaz sous pression à une deuxième évacuation de façon à ce qu'une surpression dans le dispositif d'évacuation puisse s'échapper par la deuxième évacuation dans la quatrième position du corps de vanne (3, 103).

12. Dispositif d'évacuation conformément à la revendication n°11, **caractérisé en ce que**
un deuxième conduit arrière (135) est disposé dans le corps de base (1, 101, 201) entre le troisième passage (140) et la prise (136) pour l'alimentation du gaz sous pression et/ou qu'un deuxième conduit d'évacuation (131) est disposé dans le corps de base (1, 101, 201) de manière à relier, dans la quatrième position du corps de vanne (3, 103), la dérivation du troisième passage (140) à la deuxième évacuation.

13. Dispositif d'évacuation conformément à l'une des revendications n°9 à n°10,
**caractérisé en ce que**
un filtre stérile (132, 138) est disposé sur au moins l'une des évacuations et/ou qu'un limiteur de pression est disposé dans au moins l'une des évacuations.

14. Dispositif d'évacuation conformément à l'une des revendications n°7 à n°13,
**caractérisé en ce que**
la poignée de détente (10, 110) est fermement disposée sur la partie inférieure du corps de vanne (3, 103) rotatif de telle manière à ce que le corps de vanne (3, 103) soit pivotant avec la poignée de détente (10, 110) dans le corps de base (1, 101, 201).

15. Dispositif d'évacuation conformément à l'une des revendications n°7 à n°14,
**caractérisé en ce que**
un indicateur de position (15, 115, 125) permettant la lecture de la position du corps de vanne (3, 103) dans le corps de base (1, 101, 201), en particulier sur la partie supérieure, est disposé sur le corps de vanne (3, 103) rotatif.

16. Dispositif d'évacuation conformément à l'une des revendications précédentes,
**caractérisé en ce que**
une poignée (8, 108) pour le maintien du dispositif d'évacuation est disposée sur la partie inférieure du dispositif d'évacuation.

17. Dispositif d'évacuation conformément à l'une des revendications n°7 à n°16,
**caractérisé en ce que** le corps de vanne (3, 103) est un corps de forme conique, au moins partiellement symétrique en rotation, qui est monté en rotation dans un orifice conique (2) adapté à la forme du corps de vanne (3, 103) dans le corps de base (1, 101, 201), les parois extérieures du corps de vanne (3, 103) se terminant jusqu'aux orifices des passages (140) étroitement avec les parois de l'orifice conique (2) dans le corps de base (1, 101, 201) dans chaque position du corps de vanne (3, 103) de façon à ce que les connexions des passages (140) soient près au moins d'un conduit (130, 131, 135, 137) dans le corps de base (1, 101, 201).

18. Dispositif d'évacuation conformément à la revendication n°17, **caractérisé en ce que**
un évidement (260) pour une clavette (262) est prévu dans le corps de base (1, 101, 201), une clavette (262) insérée dans l'évidement (260) pressant le corps de vanne (3, 103) dans l'orifice conique (2) dans le corps de base (1, 101, 201) de façon à ce que le corps de vanne (3, 103) génère un ajustement serré et étanche au gaz dans l'orifice conique (2).

19. Dispositif d'évacuation conformément à l'une des revendications précédentes,
**caractérisé en ce que**
une cavité pour une cartouche de gaz est prévu sur le côté détourné des pièces de jonction de la cartouche (4, 104, 204, 6, 106, 206) du dispositif d'évacuation, en particulier dans le corps de base (1, 101, 201), la prise (136) pour l'alimentation d'un gaz sous pression étant disposée dans la cavité et comprenant un mandrin (136) pour l'ouverture d'une cartouche de gaz et des moyens de fixation (146) pour la fixation d'un bouchon (17, 117, 217) avec des moyens de fixation (150) étant disposés sur le corps de base (1, 101, 201), la cartouche de gaz (142) pouvant être ouverte par la fixation du bouchon (17, 117, 217), de préférence le bouchon (17, 117, 217) comprenant en plus un moyen d'arrêt (148) en prise dans un dispositif d'arrêt (144) dans la cavité et en particulier de préférence le dispositif d'arrêt (144) positionnant une cartouche de gaz (142) insérée de taille appropriée dans la cavité.

20. Dispositif d'évacuation conformément à l'une des revendications n°9 à n°19,
**caractérisé en ce que**
les passages (140) passent essentiellement en ligne droite par l'intérieur du corps de vanne (3, 103) et que les passages (140) sont décalés les uns par rapport aux autres de 10° à 80° autour de l'axe de rotation du corps de vanne (3, 103).
